Europäisches Patentamt

(19) European Patent Office

Office européen des brevets

(11) Publication number : **0 257 755 B1**

# (12) EUROPEAN PATENT SPECIFICATION

(45) Date of publication of patent specification :
03.07.91 Bulletin 91/27

(51) Int. Cl.⁵ : **A61M 1/38, B04B 7/02**

(21) Application number : 87306082.6

(22) Date of filing : 09.07.87

(54) Centrifuge bowl or rotor for plasmapheresis.

(30) Priority : 22.07.86 US 888764

(43) Date of publication of application :
02.03.88 Bulletin 88/09

(45) Publication of the grant of the patent :
03.07.91 Bulletin 91/27

(84) Designated Contracting States :
AT BE CH DE FR GB IT LI NL SE

(56) References cited :
US-A- 4 086 924
US-A- 4 140 268
US-A- 4 300 717

(73) Proprietor : HAEMONETICS CORPORATION
400 Wood Road
Braintree Massachusetts 02184 (US)

(72) Inventor : Headley, Thomas D.
83 Westgate Road
Wellesley Massachusetts 02181 (US)

(74) Representative : Harvey, David Gareth et al
Graham Watt & Co. Riverhead
Sevenoaks Kent TN13 2BN (GB)

## Description

This invention relates to blood processing and, more specifically, plasmapheresis. More especially, the invention relates to a centrifuge bowl or rotor for plasmapheresis according to the preamble of claim 1.

Background Art

A plasmapheresis process is described in U.S. Patent 4,086,924 issued May 2, 1978 to Allen Latham, Jr. In this plasmapheresis process, whole blood is withdrawn from a donor using a phlebotomy needle and pressure cuff, is mixed with anticoagulant and is transported by a blood pump to a plasmapheresis centrifuge bowl 10 in which the whole blood is separated into a plasma and non-plasma component.

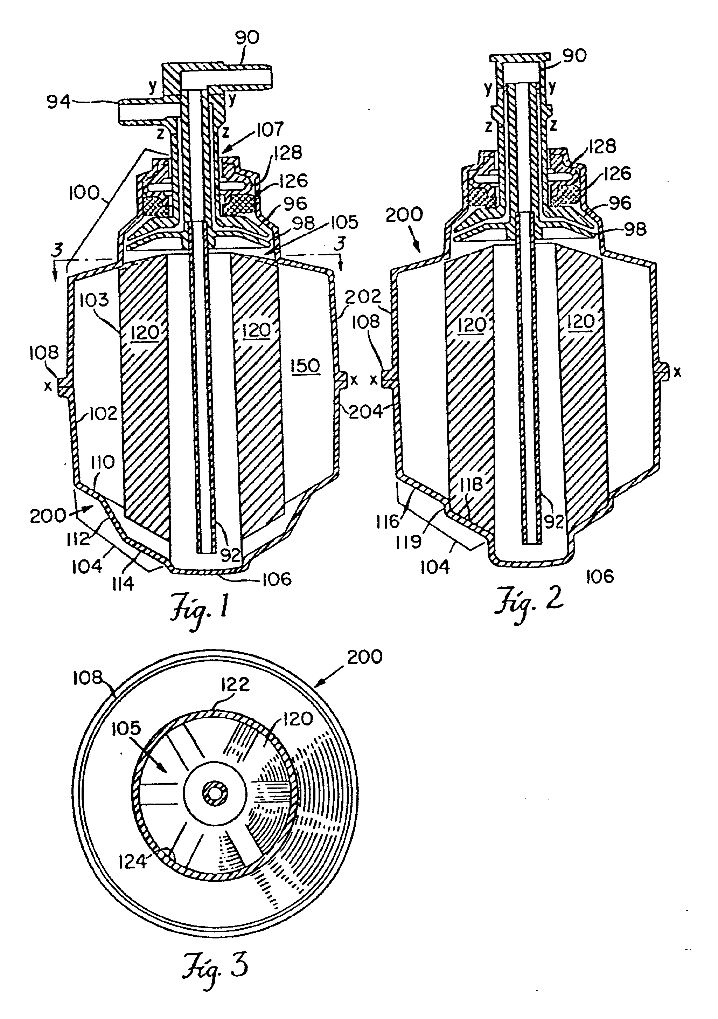

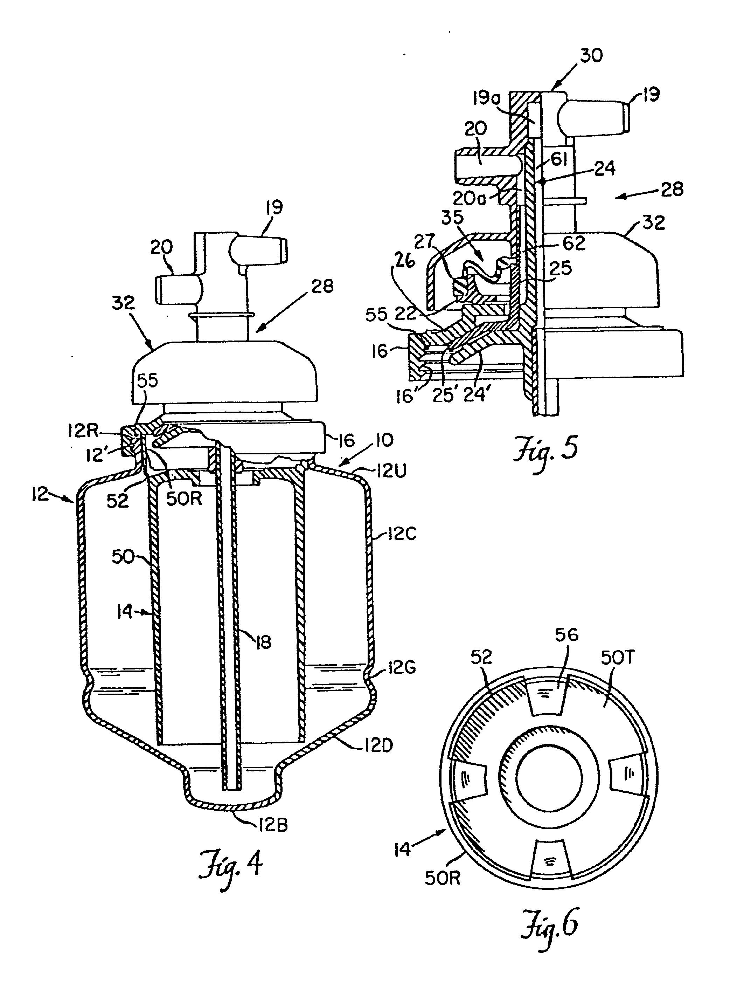

The centrifuge bowl 200 is described in connection with Figs. 4 and 5 of U.S. Patent 4,086,924, which are reproduced herein as Figs. 1, 2 and 3 and labelled "Prior Art". As shown therein, the centrifuge generally comprises a multi-piece feed tube and seal assembly with a first transverse central input port 90 leading to a longitudinal feed tube stem 92 through which whole blood is introduced into the bottom 106 of the centrifuge bowl 200.

A second transverse peripheral port 94 is provided to allow separated plasma component to flow out of the centrifuge bowl 200. A channel leading to peripheral port 94 is formed between upper skirt 96 and lower skirt 98, which extend outwardly from a point approximately opposite where the central port 90 joins feed tube stem 92.

The outlet channel formed between upper and lower skirts 96 and 98, respectively, continues into an annular coaxial channel around the periphery of the longitudinal extension of central port 90 until it joins peripheral port 94.

An elastomeric bellows 128 forms a spring member which urges carbon ring seal 126 against skirt 96 forming a rotary seal therebetween.

Central port 90, feed tube stem 92, peripheral port 94 and skirts 96 and 98 are generally manufactured by molding or extrusion techniques using blood compatible plastic, such as styrene, ABS, polycarbonate, polyethylene, polyurethane, etc. The ports and skirts are part of a feed tube assembly which is coaxial to and remains stationary while the centrifuge bowl rotates about its longitudinal axis.

The centrifuge bowl body 200 is of two piece construction formed of two integrally molded pieces, an upper piece 202 and lower piece 204, which are joined at approximately the mid-point, where a raised joint 108 is provided so that the upper and lower halves of the bowl, which were separately injection-molded, may be joined by welding. A molded series of outer wall members comprising an inclined upper wall member, shown generally at 100, a substantially vertical central wall member 102, and a lower steeply inclined wall member 104, and a recessed bottom wall member 106 define the exterior walls of the centrifuge bowl chamber.

A tubular core 120 defines a separation channel 150 between the outer core wall 103 and the inner longitudinal walls of the bowl body wall. As shown in the planar view of Fig. 3, the core is provided with vanes 122 which have steps 124 at the extremities, which are used to accurately position the core coaxially by seating part of the upper housing 124 against the vanes. When they are seated, vanes 122 serve to provide a flow channel or opening from the sedimentation area along the longitudinal length of the core wall 103, leading to the outlet channel formed between skirts 96 and 98.

Weld lines X-X, Y-Y and Z-Z have been added to Figs. 1 and 2 of the drawings to show how the individual parts and assemblies of the prior art bowl body 200 are joined together and assembled. As may be seen therefrom, the lower portion and upper portion of the feed tube and seal assembly is welded at two places, Z-Z and Y-Y, after the lower portion is inserted through a first large intermediate opening 105 and a second smaller opening 107 in upper bowl body piece 202. Next, core 120 is snapped in place on upper piece 202 by means of vanes 122 which seat on the upper piece. Finally, lower piece 204 is placed over the core 120 abutting joint 108 at X-X and is welded in place.

Apart from the overall labor intensive complexity of the above procedure, several basic problems exist in the prior art bowl construction above described. First, the weld at 108 about the periphery of the bowl body is subjected to the greatest possible centrifugal forces when the bowl is in operation since it is located at the furthest radial point from the bowl axis. Rupture of the weld at this juncture is a chief cause of bowl failure. Secondly, the bowl construction does not permit testing of the seal and feed tube assembly until it is permanently integrated with the bowl. Thus, if a defect is found in the assembly, the complete assembly must be scrapped.

Accordingly, a need exists for a more reliable, easier to assemble, centrifuge bowl capable of mass production with a minimum amount of labor and in which the seal and feed tube assembly can be separately tested.

The disposable blood processing centrifuge according to the present invention is thus characterized by comprising an integral, seamless, one-piece bowl body, and a core member which is adapted to be insertet through the aperture of said bowl prior its being covered by the seal assembly. Particular and preferred aspects of the invention are as defined in the dependent claims appended hereto.

The invention is described in more detail in the five non-limitative paragraphs which follow.

The apparatus of the present invention comprises a disposable centrifuge rotor or bowl formed of two basic items. The first is a rotary seal and header assembly ; the second, a unitary, integral, seamless, one peice bowl body. A third, optional element comprises a series of core member or members capable of being used in a standard bowl body for different separation processes. The seal and header assembly is capable of being assembled on an automatic machine. The bowl body is adapted to be manufactured by blow molding or injection blow molding and the core member, or members, by injection molding or other forming process.

The integral bowl body is adapted for rotation about its longitudinal axis. A single aperture is provided at one end of the bowl body concentric with the longitudinal axis of the bowl body. Core members of various configurations are adapted to be insertable through the aperture in the bowl body concentric with the longitudinal axis of the bowl.

The seal and header assembly has a first seal or crown member which mates with the outer body wall of the bowl member about the periphery of the bowl body aperture to affix the seal and header assembly to the bowl body. The seal and header assembly is formed of a header with input and output ports, and a feed tube coupled to the inlet port which extends within the core member concentric with the longitudinal axis of the bowl body and terminating a short distance from the lower wall of the bowl body.

An effluent tube is formed about the inner feed tube assembly with a narrow channel provided therebetween for flow of effluent, for example, plasma, to the outlet port. A second seal member is formed about the periphery of the effluent tube to form a rotary seal over the first seal member which permits the inner and outlet ports to romain stationary while the bowl rotates about its central axis.

The entire centrifuge rotor thus described can be assembled in a matter of seconds after the outside seal and header assembly has been assembled by automated assembly techniques. A selected core member is inserted into the aperture in the unitary bowl body. Next, the seal and header assembly is placed over the aperture and the feed tube inserted into a concentric opening in the core until the first seal or crown is mateably engaged with the outer wall of the aperture and a seal is formed therebetween.

The invention and preferred embodiments thereof will now be explained in further detail, by way of example only, in the following description to be read in conjunction with the accompanying drawings, in which :

Figs. 1 and 2 are respectively front and side cross-sectional views of the centrifuge bowl described in U.S. Patent 4,086,924 and labelled "Prior Art".

Fig. 3 is a transverse cross-sectional view along line 3-3 of Fig. 1 of the prior art.

Fig. 4 is a partial cutaway side cross-sectional view of the centrifuge bowl of the present invention.

Fig. 5 is a partial cut-away sectional view of a feed tube assembly of Fig. 4.

Fig. 6 is a sectional view of the top end of a core 14 seen in Fig. 4.

Fig. 6A is a perspective view of the core 14 showing the slots 52 in more detail.

Fig. 7 is a partial cutaway side cross-sectional view of the centrifuge bowl of the invention showing an alternate embodiment of the core member.

Fig. 8 is a sectional view taken along lines 8-8 of Fig. 7.

Fig. 9 is a partial cross-sectional view of yet another embodiment of the invention showing a two-piece core construction.

Fig. 10 is a detailed view taken along line 9-9 of Fig. 9.

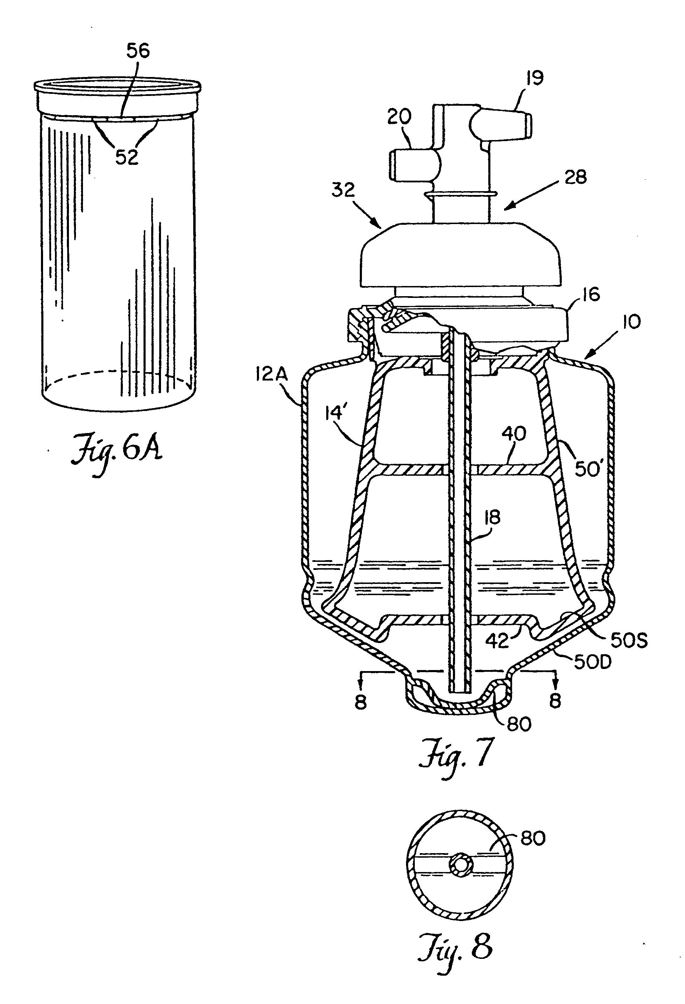

Referring now to Figs. 4-6, a preferred embodiment of the invention will now be described in connection therewith. As may be seen therein, the apparatus of the invention comprises a disposable centrifuge rotor, or bowl, 10. The bowl comprises : a seal and header assembly, shown generally at 28 (Fig. 5), a one-piece, seamless, integral bowl body shown generally at 12 (Fig. 4) and a core member 14 (Fig. 6 and Fig. 4).

The seal and header assembly 28 is capable of being assembled utilizing automatic machine techniques. The seal and header assembly is comprised of a header 30, an effluent tube 25, a feed tube assembly 24, and a rotary seal 35 formed of an upper seal ring 22, a lower seal ring 26, and a flexible member 27 and an outside seal member or crown 16.

The header 30 comprises an integral formed member having a transverse inlet bore 19 extending into a longitudinal passageway 19a coupled to the inner bore 61 of feed tube assembly 24 and, in turn, to feed tube stem 18, thus forming an inlet path for anticoagulated whole blood to enter the interior of centrifuge bowl body 12.

Header 30 also includes an outlet port, or bore 20, which extends transversely into a peripheral channel 20a extending in parallel relationship with the feed tube assembly 24 and into an outlet passageway 62. A shield portion 32 formed on header 30 extends over the rotary seal 35.

Feed tube assembly 24 is formed with an integral skirt 24' disposed adjacent a complimentary effluent tube skirt 25' formed on effluent tube 25.

The rotary seal 35, as mentioned above, is formed of a two-piece secondary seal ring which consists of flexible outside sealing member 27, which is affixed about its outer periphery to the periphery of molded ring seal 22. A seal crown 16 having internal screw threads 16', about the internal periphery thereof, is provided with a central opening through which effluent tube 25 extends. The inner periphery of flexible member 22 is joined to the effluent tube.

The header and seal assembly 28, as thus entity described, is formed and assembled as an individual entity and is inserted through an upper central opening in bowl body 12, as shown in Fig. 4 and mated with external threads 12' formed on the periphery of bowl body 12 after core member 14 has been inserted through said opening and fixed in place within the bowl body 12, as will he subsequently described.

The bowl body 12 is an integral body adapted to be manufactured by blow molding or injection blow molding and may be formed of a suitable plastic, such as transparent styrene or equivalent.

The bowl body is formed of an upper ring portion 12R, an upper diagonal portion 12U, a middle central portion 12C, a lower diagonal portion 12D and a bottom cross portion 12B. Screw threads 12' are formed on the outer surface of ring portion 12R and an opening extends longitudinally from the inner surface of the ring portion 12R into the main portion of the bowl body 12. A groove is formed about the periphery of the bowl at 12G to form a holding surface for a centrifuge rotor chuck (not shown).

An O-Ring gasket 55 is disposed on an inner peripheral shoulder of crown member 16 adjacent screw threads 16'. When member 16 is threaded onto bowl body 12, gasket 55 is compressed against the upper wall of ring 12R forming a liquid tight seal.

A cylindrical walled core 14 is adapted to be inserted into the upper opening in bowl body 12 through the opening in ring portion 12R. Core 14 is an integral member having a cylindrical outer wall 50 extending longitudinally and coaxial to the axis of bowl body 12. An upper ring portion 50R of core 14 is adapted to abut the inner wall of the ring portion 12R of bowl body 12 when the core is inserted into the upper opening of the bowl body 12.

Four L-shaped reinforcing tabs 56 are formed on the top wall 50T of core 14 to reinforce the ring portion 50R. Four peripheral slots extend along the periphery of the core 14 at the juncture between the ring portion 50R and the cylindrical wall 50, as shown more clearly in Fig. 6A. These slots 52 provide a passageway for the exit of effluent, such as plasma, which has been separated from the whole blood by the operation of the centrifuge plasma-pheresis process within the bowl body 12.

This operation may be described generally as follows : Whole blood is coupled from inlet port 19 through the longitudinal passageway in feed tube assembly 24 to the bottom of the spinning centrifuge bowl. The heavier red blood cells are forced radially outwardly from the central axis and are retained on the inner bowl wall along the main or central body portion 12C of the bowl. The lighter, less dense plasma is captured on the outer surface of cylindrical wall 50 and allowed to exit through the slots 52 at the top wall 50T of core 14 whereupon they pass through the channel between skirts 24' and 25' into the passageway 62 and out the outlet port 20 of header 30.

As mentioned previously, one of the advantages of the present invention is that the outside seal and header assembly 28 may be separately tested. For example, the assembly may be fastened to a test bowl body and checked under pressure for leaks, etc. Similarly, the bowl body 12 may be separately checked for leaks while attached to a test fixture. After testing of each, they may be assembled manually or with the use of automatic machinery.

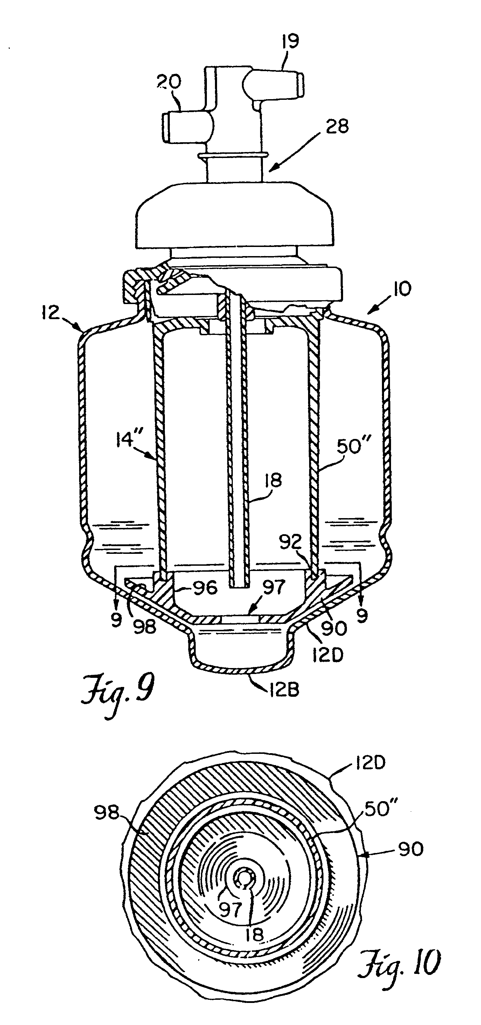

In the alternate embodiment shown in Fig. 7, the bowl body 12A and header and seal assembly 28 are substantially similar to that previously described. However, the core labelled 14' is flared at the lower extremity and is formed of semi-rigid plastic material, such as polyvinylchloride (PVC) to allow the flared core to be inserted through the upper opening in the bowl body by deflecting the longitudinal wall 50'. This results in a core body having skirts 50S extending parallel to the diagonal walls 50D of the bowl body. This skirted core configuration is similar to that shown in U.S. Patent 4,300,717 and enables different blood flow patterns to be formed within the bowl.

Fig. 8 shows the lower bowl body details of the alternate embodiment of Fig. 7 in which channels 80 are formed in the bowl body 12' during the molding process to facilitate flow of blood from the center of the bowl to the sides in a predetermined pattern.

Figs. 9 and 10 depict an alternate two-piece core embodiment in which the core member 14" is comprised of a generally cylindrical hollow walled core, as in Fig. 4, but includes a disc-like member 90 which provides a flared wall portion 98 adjacent diagonal bowl wall 12D. Member 90 is made of semi-rigid plastic which can be compressed to allow it to pass through the top opening in the bowl body. Circular grooves formed around protrusions 96 on member 90 permit cylindrical core wall 50" to mate with member 90 after the core is inserted into the bowl body. The grooves 92 form a press-fit holding the two pieces 90 and 50" together during rotation of the bowl 10. Aperture 97 permits whole blood from feed tube stem 18 to enter the bottom of the bowl body 12B.

## Equivalents

Those skilled in the art will recognize that there are many equivalents to the specific embodiments described herein. Such equivalents are intended to be encompassed within the scope of the following claims.

## Claims

1. A disposable blood processing centrifuge comprising :

a) a bowl body (12) adapted for rotation about a longitudinal axis and having a single aperture (12R) therein through an outer wall of the bowl body, which aperture is concentric with said axis; and

b) a rotary seal assembly (35) affixed to the bowl body (12) and covering the said aperture and comprising a non-rotatable upper seal ring (22) and a lower rotatable seal ring (26) ;

c) a core member (14, 14', 14") ;

d) and a non-rotational header 30 with input and output ports (19), (20) affixed thereto, characterized in that the bowl body (12) is an integral, seamless, one-piece bowl body and the core member (14, 14', 14") is adapted to be inserted through the said aperture (12R) prior its being covered by the seal assembly.

2. The centrifuge according to claim 1, wherein the core member (14') is semi-rigid and flared at one end.

3. The centrifuge according to claim 2, wherein the core member (14") comprises a rigid member (50") having an outer diameter small enough to enable passage through the said aperture and a flexible member (90) having a greater diameter which is compressed to pass through the said aperture and thereafter expanded and affixed to the rigid member.

4. The centrifuge according to claim 1, 2 or 3 wherein the rotary seal assembly (35) is provided with a threaded cover (16) which is screwed onto complementary threads (12') on the bowl body (12) to cover the aperture thereof.

5. The centrifuge according to claim 4, wherein a crown 16 is disposed between the lower seal ring (26) and bowl body (12) about the periphery of the aperture.

6. The centrifuge according to any of claims 1 to 5, wherein the bowl body has shaped portions thereon, as formed, which affect the flow of blood components.

7. The centrifuge according to claim 6, wherein the core member (14', 14") is at least partly flexible at portions thereof having an outer diameter greater than the bowl body aperture.

8. The centrifuge according to claim 7 including a peripheral opening extending at least partially about the periphery of said core member for permitting exit of separated whole blood constituents from said bowl body to said effluent port.

## Ansprüche

1. Einwegzentrifuge zur Blutbehandlung, bestehend aus

a) einem Gehäusekörper (12), der für eine Drehung um eine Längsachse eingerichtet ist und eine einzelne Öffnung (12R) durch eine Außenwand des Gehäusekörpers aufweist, welche Öffnung konzentrisch zu der Achse ist, und

b) einer Drehdichtung (35), die am Gehäusekörper (12) festgelegt ist, die Öffnung abdeckt und einen nicht-drehbaren oberen Dichtungsring (22) und einen unteren drehbaren Dichtungsring (26) umfaßt,

c) einem Kernteil (14, 14',14") und

d) einem nicht-drehbaren Kopfstück (30) mit daran angebrachtem Eingangs- und Ausgangskanal (19, 20), dadurch gekennzeichnet, daß der Gehäusekörper (12) ein einheitlicher, nahtloser, einstückiger Gehäusekörper ist und das Kernteil (14, 14', 14") durch die Öffnung (12R) vor ihrer Abdeckung durch die Dichtung einsetzbar ist.

2. Zentrifuge nach Anspruch 1, bei der das Kernteil (14') halbhart und an einem Ende konisch erweitert ist.

3. Zentrifuge nach Anspruch 2, bei der das Kernteil (14") ein hartes Teil (50") mit einem so kleinen Außendurchmesser, daß ein Durchgang durch die Öffnung möglich ist, und ein flexibles Teil (90) mit einem größeren Durchmesser umfaßt, das für einen Durchgang durch die Öffnung zusammengedrückt und danach aufgeweitet und an dem harten Teil festgelegt wird.

4. Zentrifuge nach Anspruch 1, 2 oder 3, bei der die Drehdichtung (35) mit einem Gewindedeckel (16) versehen ist, der auf ein Gegengewinde (12') am Gehäusekörper (12) zur Abdeckung dessen Öffnung aufgeschraubt ist.

5. Zentrifuge nach Anspruch 4, bei der eine Kappe (16) zwischen dem unteren Dichtungsring (26) und dem Gehäusekörper (12) über den Umfang der Öffnung angeordnet ist.

6. Zentrifuge nach einem der Ansprüche 1 bis 5, bei der der Gehäusekörper Formteile, wie ausgeformt, besitzt, die die Strömung der Blutkomponenten beeinflussen.

7. Zentrifuge nach Anspruch 6, bei der das Kernteil (14',14") zumindest teilweise flexibel in Bereichen ist, die einen Außendurchmesser, der größer als die Gehäusekörperöffnung ist, aufweisen.

8. Zentrifuge nach Anspruch 7, mit einer Umfangsöffnung, die sich zumindest teilweise um den Umfang des Kernteils erstreckt, um ein Austreten von vom Gesamtblut abgetrennten Bestandteilen aus dem Gehäusekörper zum Ablaufkanal zu ermöglichen.

## Revendications

1. Bol de centrifugation jetable pour le traitement du sang comprenant :

    a) un corps de bol (12) adapté de manière à pouvoir tourner autour d'un axe longitudinal et ayant une seule ouverture (12R) à l'intérieur, à travers une paroi externe du corps de bol, cette ouverture étant concentrique par rapport à l'axe ; et

    b) un ensemble de joint d'étanchéité rotatif (35) fixé au corps de bol (12) et recouvrant ladite ouverture, cet ensemble comprenant un anneau d'étanchéité supérieur non rotatif (22) et un anneau d'étanchéité inférieur rotatif (26) ;

    c) un noyau (14, 14', 14") ;

    d) et un chapeau non rotatif (30) avec des orifices d'entrée et de sortie (19, 20) fixés à ce chapeau, caractérisé en ce que le corps de bol (12) est un corps de bol intégral, d'une seule pièce, sans joint de soudure et le noyau (14, 14', 14") est adapté de manière à pouvoir être introduit à travers ladite ouverture (12R) avant qu'elle ne soit recouverte par l'ensemble de joint d'étanchéité.

2. Bol de centrifugation suivant la revendication 1 caractérisé en ce que le noyau (14') est semi-rigide et il est évasé à une extrémité.

3. Bol de centrifugation suivant la revendication 2 caractérisé en ce que le noyau (14") comprend un élément rigide (50") ayant un diamètre externe suffisamment petit pour permettre son passage à travers ladite ouverture, et un élément flexible (90) ayant un diamètre plus grand, lequel est comprimé pour pouvoir passer à travers ladite ouverture et se dilater ensuite, cet élément flexible étant fixé à l'élément rigide.

4. Bol de centrifugation suivant la revendication 1, 2 ou 3 caractérisé en ce que l'ensemble de joint d'étanchéité rotatif (35) est pourvu d'un couvercle fileté (16) qui est vissé sur un filetage complémentaire (12') prévu sur le corps de bol (12), afin de recouvrir l'ouverture de celui-ci.

5. Bol de centrifugation suivant la revendication 4 caractérisé en ce qu'une couronne (16) est disposée entre l'anneau d'étanchéité inférieur (26) et le corps de bol (12), autour de la périphérie de l'ouverture.

6. Bol de centrifugation suivant l'une quelconque des revendications 1 à 5 caractérisé en ce que le corps de bol est formé avec des parties façonnées sur lui, lesquelles affectent l'écoulement des composants du sang.

7. Bol de centrifugation suivant la revendication 6 caractérisé en ce que le noyau (14', 14") est flexible, au moins partiellement, à l'endroit de parties de celui-ci ayant un diamètre externe supérieur à l'ouverture du corps de bol.

8. Bol de centrifugation suivant la revendication 7 caractérisé en ce qu'il comporte une ouverture périphérique s'étendant au moins partiellement autour de la périphérie du noyau, afin de permettre la sortie, à partir du corps de bol et en direction de l'orifice d'effluent, de parties séparées du sang complet.

*Fig. 1*

*Fig. 2*

*Fig. 3*

*Fig. 4*

*Fig. 5*

*Fig. 6*

*Fig. 6A*

*Fig. 7*

*Fig. 8*

*Fig. 9*

*Fig. 10*